# EUROPEAN PATENT SPECIFICATION

(11) **EP 3 092 942 B1**
(45) Date of publication and mention of the grant of the patent: **22.07.2020**
(21) Application number: 16169181.1
(22) Date of filing: 11.05.2016
(51) Int. Cl.: A61B 5/0402, A61B 5/0408, A61B 5/00

(54) **CUSTOMIZABLE ELECTROPHYSIOLOGICAL MAPPING ELECTRODE PATCH AND METHOD OF CONFIGURATION**
INDIVIDUELL ANPASSBARES ELEKTROPHYSIOLOGISCHES MAPPING-ELEKTRODENPATCH UND VERFAHREN ZUR DESSEN KONFIGURATION
PATCH ÉLECTROPHYSIOLOGIQUE DE CARTOGRAPHIE PERSONNALISABLE ET MÉTHODE DE CONFIGURATION

(30) Priority: 13.05.2015 US 201562161065 P; 01.05.2016 US 201615143610
(43) Date of publication of application: 16.11.2016
(73) Proprietor: EP Solutions SA, 1400 Yverdon-les-Bains (CH)
(72) Inventor: CAILLER, Yann, 1071 Chexbres (CH); HALLER, Markus, 1260 Nyon (CH)
(74) Representative: Studio Torta S.p.A.

(56) References cited:
- EP-A2- 0 509 689
- WO-A1-2010/054352
- US-A- 4 498 480
- US-B1- 6 847 836

## Description

### Field of the Invention

Various embodiments described herein relate to the field of electrophysiological mapping and treatment medical systems, devices, components, and methods.

### Background

Non-invasive electrophysiological sensing and mapping of a patient's cardiac or other electrical activity inside the patient typically requires the simultaneous acquisition of numerous surface electrocardiograms (ECGs) using sensing electrodes positioned at many different locations on a patient's torso. The positioning and attachment of such sensing electrodes can be a laborious and time consuming process. In addition, if electrophysiological mapping is to be carried out in combination with other medical procedures, such as navigation of a medical device inside a patient's body, or cardiac or other organ ablation procedures, cardio-version patches, navigation patches (such as Carto3® patches) and ultrasound transducers may also need to be used (in addition to sensing electrodes). Such patches and transducers often must be positioned in between sensing electrodes, which can pose additional difficulties and consume valuable hospital or clinic time.

What is needed are more efficient and quicker means and methods for acquiring ECGs, as well as means and methods for more efficient and quicker acquisition of ECGs in combination with other types of medical procedures.

WO 2010/054352 A1 discloses a sensor array for sensing electrical activity of a patient; the sensor array includes a plurality of elongate strips of a flexible substrate material, a plurality of electrically conducive sensors distributed along the elongated strips to provide a column of sensors along each respective strip. Connecting elements maintain a desired relative position of the sensors; one or more of the connecting elements can be broken, in order to separate the strips and allow repositioning of the associated sensors.

US 6 847 836 B1 discloses an ECG electrode chest pad having an upper portion with upper limb electrodes; a central portion with a plurality of precordal unipolar electrodes and a lower portion with lower limb electrodes. The pad has a perforation in the base pad material such that one group of electrodes may be removed from the sensor pad, if the electrodes are not used for monitoring.

EP 0 509 689 A2 discloses an electrode strip for use in electrocardiography comprising a flexible and substantially inextensible substrate, a plurality of conductors that extend along the substrate to form a plurality of electrode sites, and a cover layer that insulates the conductors. A plurality of regions of extensibility in the strip allow selective positioning of the electrodes sites on a body.

### Summary

In one embodiment, there is provided a customizable electrophysiological mapping sensor patch configured for placement on and attachment to a patient's torso, where the patch comprises a first substantially planar layer comprising at least a first flexible material, a second substantially planar electrically insulative layer comprising at least a second flexible material, a plurality of flexible electrical conductors disposed on or within the first layer, or disposed at least partially between the first layer and the second layer, each of the flexible electrical conductors having at least first proximal and second distal ends, a plurality of sensing electrodes, each sensing electrode being operably connected to one of the electrical conductors, a plurality of adhesive members, each adhesive member surrounding at least partially a corresponding one or more of the sensing electrodes, and at least one score line disposed in the mapping sensor patch, the score line defining at least one pre-cut portion of the mapping sensor patch, the score line being configured to permit a user to remove or partially remove the at least one pre-cut portion from the mapping sensor patch by pulling or cutting the mapping sensor patch or pre-cut portion along or near the score line. The customizable electrophysiological mapping sensor patch may further comprise a plurality of position markers, each position marker having a position corresponding to that of a selected one of the sensing electrodes.

At least one pre-cut portion of the patch may comprise one or more of a row pre-cut, a column pre-cut, a cardioversion patch pre-cut, a navigation patch pre-cut, a ground patch pre-cut, a female anatomy customization pre-cut, an actuator pre-cut, a sensor pre-cut, or an anatomical reference mark pre-cut.
At least one of the first layer and the second layer may comprise a woven fabric. At least some of the first proximal ends of the electrical conductors may be shaped and configured for attachment to external mapping cable electrical connectors.
At least some of the electrical conductors may comprise a screen printed electrically conductive material disposed upon the first layer or the second layer, or an electrically conductive fabric or yarn disposed upon or within the first layer or the second layer.
At least some of the sensing electrodes may comprise a metal or metal alloy member, an electrically conductive yarn or fiber, a screen printed electrically conductive material, or a screen printed electrically conductive material incorporated into or forming a portion of the distal end of the electrical conductor corresponding thereto.
At least some of the sensing electrodes may comprise an electrically conductive gel.
The customizable electrophysiological mapping sensor patch may further comprise additional electrodes not attached to the first layer or second layer and configured for placement on one or more of a patient's breasts.
At least some of the sensing electrodes may comprise one or more magnetic resonance imaging (MRI), computer tomography (CT), and ultrasound contrast agents.
The customizable electrophysiological mapping sensor patch may further comprise a protective layer configured to cover at least the adhesive members, the protective layer being configured to be removed from the mapping sensor patch by a user before the mapping sensor patch is applied to the patient's torso.
The patch may preferably be a front patch and has a width ranging between about 260 mm and about 320 mm, a length ranging between about 200 mm and about 250 mm, and further comprises between 50 and 90 electrodes arranged in between 7 and 11 columns.

The patch may preferably be a back patch and has a width ranging between about 300 mm and about 340 mm, a length ranging between about 200 mm and about 250 mm, and further comprises shoulder straps and between 50 and 90 electrodes arranged in between 8 and 12 columns. Preferably, the patch may be a side patch and has a width ranging between about 50 mm and about 350 mm, a length ranging between about 200 mm and about 250 mm, and further comprises between 10 and 90 electrodes arranged in between 1 and 12 columns.

In another embodiment, there is provided a system of customizable electrophysiological mapping sensor patches configured for placement on and attachment to a patient's torso, where the system comprises a front mapping sensor patch, a rear mapping sensor patch, and two side mapping sensor patches, each of the patches comprising a first substantially planar layer comprising at least a first flexible material, a second substantially planar electrically insulative layer comprising at least a second flexible material, a plurality of flexible electrical conductors disposed on or within the first layer, or disposed at least partially between the first layer and the second layer, each of the flexible electrical conductors having at least first proximal and second distal ends, a plurality of sensing electrodes, each sensing electrode being operably connected to one of the electrical conductors, a plurality of adhesive members, each adhesive member surrounding at least partially a corresponding one or more of the sensing electrodes, , and at least one score line disposed in the mapping sensor patch, the score line being configured to permit a user to remove or partially remove at least one pre-cut portion from the mapping sensor patch by pulling or cutting the mapping sensor patch or pre-cut portion along or near the score line.

The system of customizable electrophysiological mapping sensor patches may further comprise a plurality of position markers, each position marker having a position corresponding to that of a selected one of the sensing electrodes.
At least one pre-cut portion of each patch may comprise one or more of a row pre-cut, a column pre-cut, a cardioversion patch pre-cut, a navigation patch pre-cut, a ground patch pre-cut, a female anatomy customization pre-cut, an actuator pre-cut, a sensor pre-cut, or an anatomical reference mark pre-cut.
At least some of the sensing electrodes may comprise a metal or metal alloy member, an electrically conductive yarn or fiber, a screen printed electrically conductive material, or a screen printed electrically conductive material incorporated into or forming a portion of the distal end of the electrical conductor corresponding thereto.
Each patch may further comprise a protective layer configured to cover at least the adhesive members, the protective layer being configured to be removed from each mapping sensor patch by a user before the mapping sensor patch is applied to the patient's torso.
The front patch may preferably have a width ranging between about 260 mm and about 320 mm, a length ranging between about 200 mm and about 250 mm, and further comprises between 50 and 90 electrodes arranged in between 7 and 11 columns, the back patch has a width ranging between about 300 mm and about 340 mm, a length ranging between about 200 mm and about 250 mm, and further comprises shoulder straps and between 50 and 90 electrodes arranged in between 8 and 12 columns, and each side patch and has a width ranging between about 50 mm and about 350 mm, a length ranging between about 200 mm and about 250 mm, and further comprises between 10 and 90 electrodes arranged in between 1 and 12 columns.

In still another embodiment, there is provided a method of configuring a customizable electrophysiological mapping sensor patch for a patient's torso, the mapping sensor patch comprising a first layer comprising at least a first flexible material, a second electrically insulative layer comprising at least a second flexible material, a plurality of flexible electrical conductors disposed on or within the first layer, or disposed at least partially between the first layer and the second layer, each of the flexible electrical conductors having at least first proximal and second distal ends, a plurality of sensing electrodes, each sensing electrode being operably connected to one of the electrical conductors, a plurality of adhesive members, each adhesive member surrounding at least partially a corresponding one or more of the sensing electrodes, at least one score line disposed in the mapping sensor patch, the score line defining at least one pre-cut portion of the mapping sensor patch, the score line being configured to permit a user to remove or partially remove the at least one pre-cut portion from the mapping sensor patch by pulling or cutting the mapping sensor patch or pre-cut portion along or near the score line, where the method comprises removing at least one of the pre-cut portions from the mapping sensor patch on the basis of a procedure that is to be performed on the patient and on the basis of the patient's torso anatomy or torso dimensions.

Further embodiments are disclosed herein or will become apparent to those skilled in the art after having read and understood the specification and drawings hereof.

### Brief Description of the Drawings

Different aspects of the various embodiments will become apparent from the following specification, drawings and claims in which:
Fig. 1 shows one embodiment of a basic method and system for non-invasive electrophysiological mapping of a patient's heart activity;
Figs. 2A through 2E show various devices and components associated with one embodiment of mapping electrode system 100;
Figs. 3A through 3C show, respectively, one embodiment of customizable electrophysiological mapping sensor front patch 101, one embodiment of customizable electrophysiological mapping sensor side patch 103a, and one embodiment of customizable electrophysiological mapping sensor back patch 105 mounted on or affixed to torso 14 of patient 12;
Figs. 4A through 4C show top plan, side exploded, and cross-sectional views, respectively of one embodiment of a portion of a customizable electrophysiological mapping sensor patch;
Fig. 5A shows one embodiment of a customizable electrophysiological mapping sensor front patch 101;
Fig. 5B shows one embodiment of a customizable electrophysiological mapping sensor side patch 103;
Fig. 5C shows one embodiment of a customizable electrophysiological mapping sensor rear patch 105;
Fig. 6A shows one embodiment of a customizable electrophysiological mapping sensor front patch 101 having no top or first layer 112 disposed thereon, and
Fig. 6B shows one embodiment of a customizable electrophysiological mapping sensor patch system comprising front patch 101, side patches 103A and 103B, and rear patch 105 operably connected to data acquisition device 210 through ECG mapping cables 102a through 102d.

The drawings are not necessarily to scale. Like numbers refer to like parts or steps throughout the drawings.

### Detailed Descriptions of Some Embodiments

Described herein are various embodiments of systems, devices, components and methods for conducting electrophysiological studies.

Technical features described in the context of a specific embodiment may be combined with the features of other described embodiments unless the contrary is explicitly stated.

At least portions or components of the EP Solutions 01C System for Non-Invasive Cardiac Electrophysiology Studies (which is based upon and in most aspects the same as the AMYCARD 01 C System for Non-Invasive Cardiac Electrophysiology Studies) may be adapted for use in conjunction with the various embodiments described and disclosed herein. Portions of the EP Solutions 01C System (hereafter "the EP Solutions 01C System") and other relevant components, devices and methods are described in:
(a) U.S. Patent No. 8,388,547 to Revishvili et al. entitled "Method of Noninvasive Electrophysiological Study of the Heart" ("the '547 patent");
(b) U.S. Patent No. 8,529,461 to Revishvili et al. entitled "Method of Noninvasive Electrophysiological Study of the Heart" ("the '461 patent"), and (c) U.S. Patent No. 8,660,639 to Revishvili et al. entitled "Method of Noninvasive Electrophysiological Study of the Heart" ("the '639 patent").

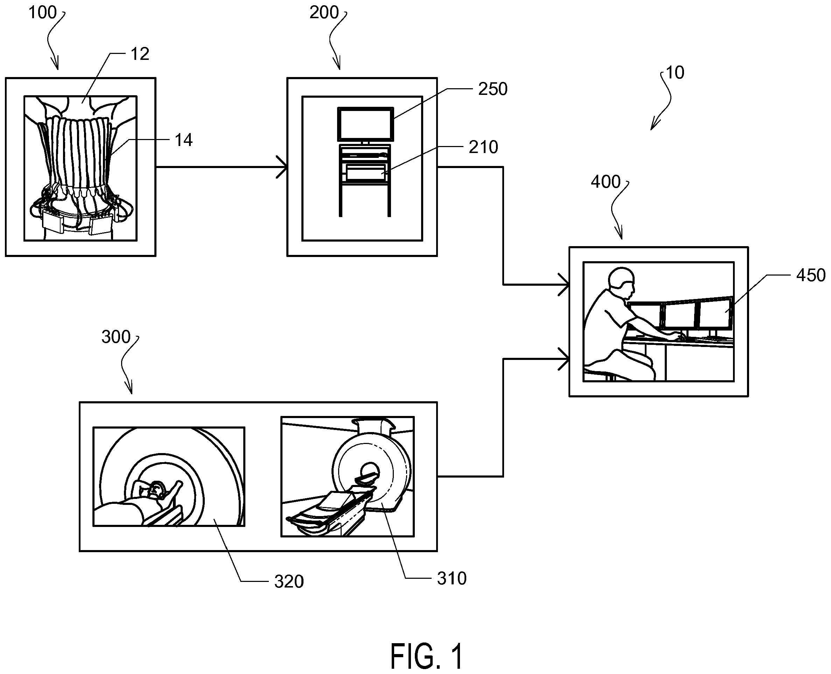

Referring now to Fig. 1, there is shown one embodiment of a basic method and system 10 for non-invasive electrophysiological mapping of a patient's heart activity. As shown, electrophysiological mapping system 10 ("EPM 10") comprises four basic sub-systems: (a) mapping electrode system 100 ("MES 100") disposed on patient 12's torso 14; (b) multichannel mapping unit 200 ("MMU 200"), which in one embodiment comprises a data acquisition device 210 and a corresponding first computer workstation 250 for multichannel mapping of the heart; (c) scanner or imaging system 300, which in one embodiment is a computed tomography scanner 310 or an MRI scanner 320 (although under certain circumstances other suitable types of medical imaging devices and systems may also be used, such as ultrasound imaging systems or fluoroscopy imaging systems); and (d) processing and visualization module 400 ("PVM 400"), which in one embodiment comprises a second computer workstation 450. (Note that in some embodiments the first computer workstation 250 of MMU 200 and the second computer workstation 450 of PVM 400 may be combined into a single computer workstation, may comprise more than two computers or computer workstations, and/or may include computing capability and/or storage provided by a network of local or remote computers, servers, or the cloud.)

Mapping electrode system 100 comprises a plurality of electrical sensing electrodes positioned on a torso 14 of patient 12 (and in some embodiments on other portions of patient 12's body). Sensing electrodes in MES 100 are configured to sense electrical activity originating in patient 12's body. In addition to electrical sensing electrodes, other types of devices and/or transducers, such as ground electrodes, high intensity focused ultrasound transducers, ultrasound probes, navigation patches, cardioversion patches, and the like (more about which is said below), may be configured to operate in conjunction with, be incorporated into, or form a portion of MES 100 and/or system 10. In one embodiment, and by way of non-limiting illustrative example, MES 100 comprises one or more of an ECG cable with 12 leads and corresponding electrodes, an ECG cable with 4 leads and corresponding electrodes, a patient cable for ECG-mapping with 8 contacts or electrodes, one or more special ECG-mapping cables (each with 56 contacts or electrodes), and special disposable or reusable mapping electrodes, each strip of disposable or reusable mapping electrodes having 8 contacts or electrodes. One example of a disposable ECG electrode suitable for use in CT imaging systems is the Model No. DE-CT electrode manufactured by EP Solutions SA, Rue Galilee 7, CH-1400 Yverdon-les-Bains. Other types and models of electrodes are, of course contemplated, such as electrodes configured for use with MRT/MRI imaging systems or other types of imaging systems 300.

Scanner or imaging system 300 is used to help identify and determine the precise positions of the various electrodes included in MES 100 that have been placed in various positions and locations on patient 12's body.

ECG data are acquired from MES 100 by MMU 200, which in one embodiment comprises an amplifier unit or data acquisition device 210 that filters and amplifies analog signals provided by MES 100, digitizes such analog signals using one or more analog-to-digital converters ("ADCs"), and sends or relays, or otherwise transfers or has transferred, the amplified and digitized signals to first computer workstation 250. In one embodiment, amplifier unit or data acquisition device 210 permits multichannel synchronous EKG/ECG recording from, by way of example, 224 or more surface electrodes positioned on a patient's skin and torso, as well as multichannel synchronous EKG/ECG recording from additional or other electrodes or channels (as described above in connection with MES 100).

In one embodiment, first computer or computer workstation 250 stores or records the amplified and digitized signals provided by data acquisition device 210. Signal digitization and recording functions can also be apportioned or split between data acquisition device 210 and first computer or computer workstation 250, as suitable or desired. Data from scanner 300 and ECG data sensed by MES 100 and acquired and recorded by MMU 200 are then both input into PVM 400. In one embodiment, ECG data from patient 12 are acquired using MES 100 and data acquisition device 210 from unipolar electrodes positioned on patient's torso 14. The precise locations of such electrodes on patient's torso 14 are determined in PVM 400 using data from scanner 300. ECG data recorded by MMU 200 may be stored on a CD, a USB memory stick, in RAM, on an electronic storage device such as a hard or flash drive, or in another memory device or component, and may then be exported or transferred to PVM 400 using such a storage device. Alternatively, ECG data recorded by MMU 200 may be transferred to PVM 400, by way of non-limiting illustrative example, using a local area network (LAN), a wide area network (WAN), wireless communication means (e.g., using Bluetooth or the Medical Implant Communication System or MICS), the internet or the cloud, or by suitable computer communication means known to those skilled in the art. In imaging system 300 and/or in PVM 400 (but typically in imaging system 300), computed tomography or other types of positional/spatial geometry data corresponding to the chest and heart area is carried out, and processing and analysis of multichannel ECG data and computed tomography or other positional/spatial geometry data are executed.

By way of non-limiting illustrative example, PVM 400 comprises a second computer or computer workstation 450 that comprises a specialized processing and visualization computer or series of interconnected computers or processors, which include pre-installed proprietary software configured to conduct electrophysiological studies. Second computer or computer workstation 450 typically further comprises a keyboard a mouse, a display 414 (such as a 24" or 25" LCD monitor), and a printer. PVM 400 and second computer workstation 450 are configured for advanced mathematical processing of computed tomographic study or other positional/spatial geometry data combined with multichannel ECG body surface mapping data, which together make it possible to perform computed non-invasive activation mapping of the patient's heart.

Together, MES 100, MMU 200, scanner 300 and PVM 400 comprise EPM 10 and employ a technique known as NIEM (Non-Invasive Electrophysiological Mapping), which is an electrophysiological method based on non-invasive reconstruction of cardiac activation patterns sensed by a dense network of surface electrodes attached to the patient's torso. NIEM is employed in EPM 10 to permit non-invasive numerical reconstruction of endocardial as well as epicardial and/or pericardial electrograms originating from the patient's ventricles and atria. Mathematical algorithms executed by EPM 10 are applied to the acquired unipolar surface ECG data to permit 3D reconstruction of the patient's heart and thorax.

In one embodiment, EPM 10 reconstructs electrograms using advanced tomographic techniques that eliminate the need to perform invasive mapping studies or procedures on the patient's body. Based on surface electrograms or ECGs acquired on the patient's torso, time-varying electric field potentials of the patient's heart are calculated using electrical field calculation techniques and algorithms. Actual boundaries of the patient's chest and lung surfaces, and of the patient's epicardial and endocardial heart surfaces, are determined by user interaction and image processing algorithms (such as solving differential equation systems). Continuations of electric field potentials throughout the patient's chest surfaces are implemented computationally based on a solution of the Cauchy problem for the Laplace equation in a homogeneous or inhomogeneous medium. When solving the Cauchy problem using the Laplace equation, a model of the chest is employed having tissues that lie within the bounds of large anatomic structures (e.g., the lungs, mediastinum, and/or spine), and that have constant or tissue-specific coefficients of electrical conductivity. Heart electric field potentials are assigned harmonic functions in each region, where each region has a constant coefficient of electrical conductivity and satisfies conjugate conditions at the region's borders for electrical potential and current.

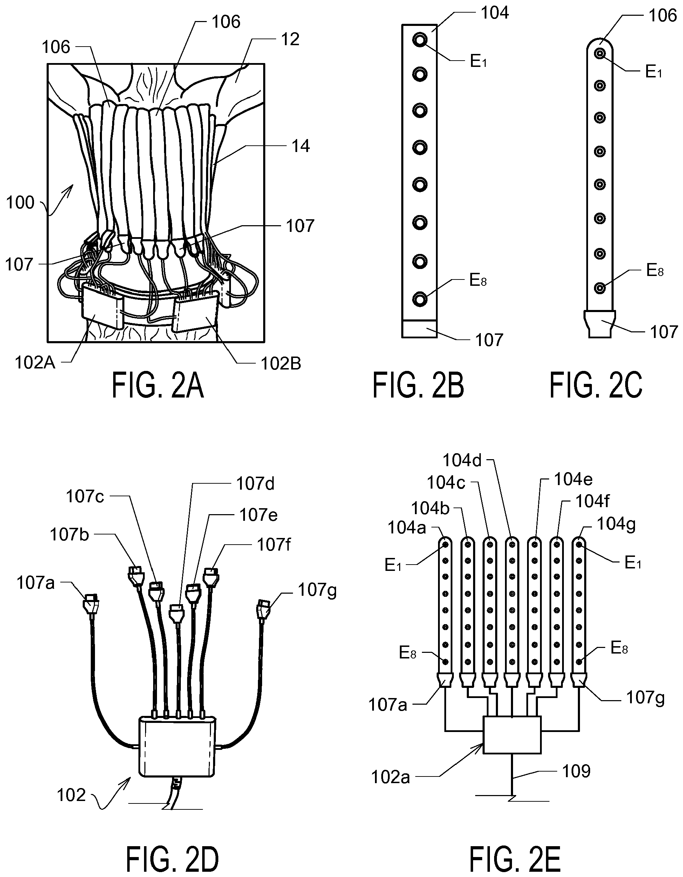

Figs. 2A through 2E show various devices and components associated with one embodiment of mapping electrode system 100 (or MES 100, as described above).

FIG 2A shows a front vie w of patient 12 having strips of electrodes affixed to flat patient cables 106, where flat patient cables 106 are attached or adhered to patient's torso 14, generally by means of a biocompatible adhesive disposed on the lower surfaces of cables 106, where the adhesive is configured to permit easy removal of cables 106 from patient's torso 14 after the electrophysiological mapping procedure has been completed. In one embodiment, flat patient cables 106 (or disposable electrode strips 104 - see Fig. 2B) comprise 8 electrodes E₁ through E₈ each, and six flat patient cables 106 or disposable electrode strips 104 are attached to each ECG mapping cable 102 by means of mapping cable electrode connectors 107.

Fig. 2B shows one embodiment of a disposable electrode strip 104, which comprises 8 electrodes E₁ through Es, and also comprises on its lower surface a biocompatible adhesive that permits easy removal of electrode strip 104 from patient's torso 14 after the electrophysiological mapping procedure has been completed. Disposable electrode strip 104 may also comprise mapping cable electrode connectors 107, or electrical connections may be established directly to each of electrodes E₁ through E₈ by means of separate electrical connections.

Fig. 2C shows one embodiment of a flat patient cable 106, which comprises 8 electrodes E₁ through E₈, and also comprises on its lower surface a biocompatible adhesive that permits easy removal of electrode strip 106 from patient's torso 14 after the electrophysiological mapping procedure has been completed. Flat patient cable 106 may also comprise mapping cable electrode connectors 107, or electrical connections may be established directly to each of electrodes E₁ through E₈ by means of separate electrical connections.
Fig. 2D shows one embodiment of an ECG mapping cable 102, which is configured to permit operable electrical connection thereto of seven separate disposable electrode strips 104 or seven flat patient cables 106 via mapping cable electrode connectors 107a through 107g. Mapping cable data acquisition module connectors 109 of ECG mapping cable 102 are configured for attachment to corresponding electrical connectors disposed in data acquisition device 210.

Fig. 2E shows one embodiment of an ECG mapping cable 102 operably connected to seven separate disposable electrode strips 104 or seven flat patient cables 106, each containing 8 electrodes E₁ through E₈ via mapping cable electrode connectors 107a through 107g. In addition to performing the above mentioned tasks, ease of use, patient comfort, patient's morphology adaptiveness and noise reduction are important features that should be satisfied.

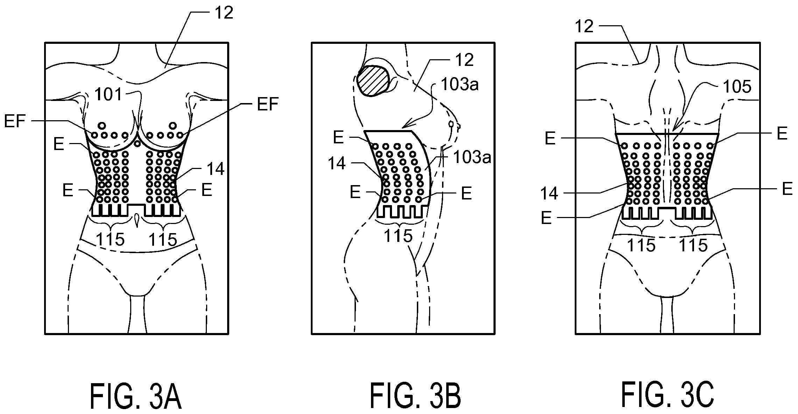

Referring now to Figs. 3A though 6B, there are shown various embodiments of patches 101, 103 and 105 that provide solutions to various problems existing in the prior art respecting electrophysiological mapping and studies. Some embodiments of patches 101, 103 and 105 permit body surface ECG signal acquisition to be performed quickly and easily, and also to be combined quickly and easily with non-invasive mapping and navigation tools, cardioversion techniques, and invasive and non-invasive ablation methods. As will become apparent to those skilled in the art upon having read and understood the present specification and claims, patches 101, 103 and105 increase the efficiency and reduce the time required to carry out electrophysiological studies and mapping, increase patient comfort, are easily adaptable to changes in patient morphology, reduce ECG sensor noise, and may be combined easily with at least some other medical sensing and treatment procedures.

Figs. 3A through 3C show, respectively, one embodiment of customizable electrophysiological mapping sensor front patch 101, one embodiment of customizable electrophysiological mapping sensor side patch 103a, and one embodiment of customizable electrophysiological mapping sensor back patch 105 mounted on, adhered or otherwise affixed to torso 14 of patient 12. As shown in Figs. 3A through 3C, each of patches 101, 103a and 105 comprises a plurality of sensing electrodes E, which in one embodiment are unipolar electrodes integrated into a fabric or other flexible material(s) from which each of patches 101, 103a and 105 is formed (more about which is said below). Rather than attach a plurality of individual electrode strips 104 or patient cables 106 to patient's torso 14, it will be seen that patches 101, 103a (and 103b - not shown in Figs. 3A through 3C), and 105 are considerably less labor intensive and time consuming to place on patient 12. In Figs. 3A through 3C, proximal electrical connections 115 are configured for attachment to corresponding ECG mapping cable connectors 107, or to any other suitable electrical connector configured to convey electrical signals generated by sensing electrodes E to data acquisition device 210.

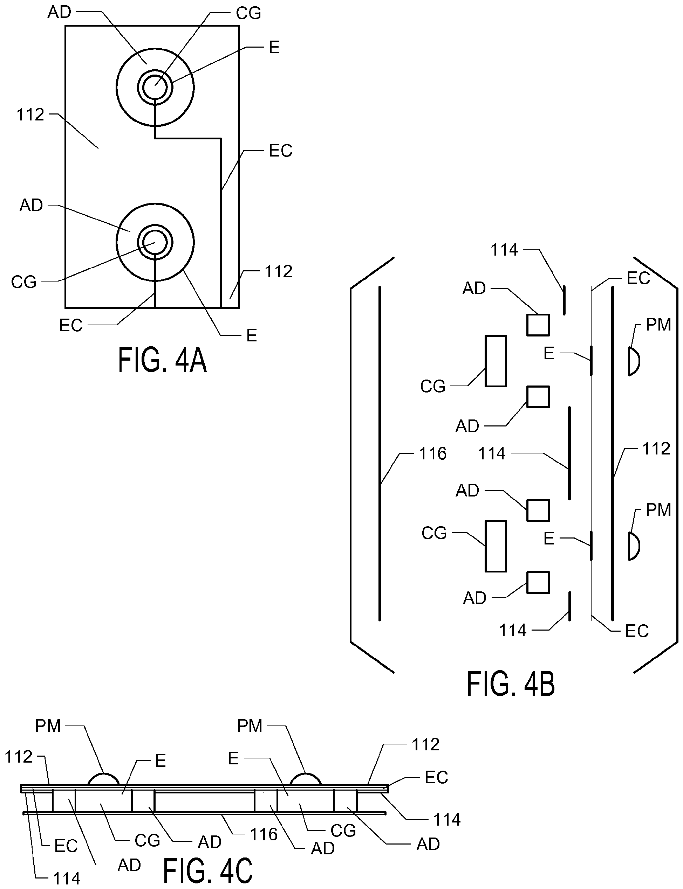

Figs. 4A through 4C show top plan, side exploded, and cross-sectional views, respectively of one embodiment of a small illustrative portion of a customizable electrophysiological mapping sensor patch configured for placement on and attachment to a patient's torso 14. In Figs. 4A, 4B and 4C, first or upper substantially planar layer 112 comprises at least a first flexible material, while second or lower substantially planar electrically insulative layer 116 comprises at least a second flexible material.

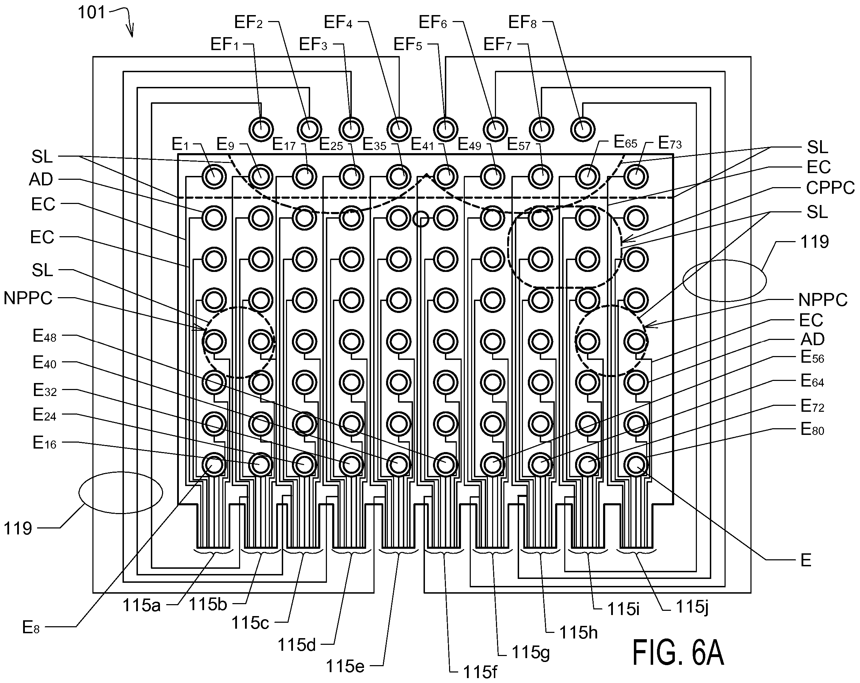

In one embodiment, a plurality of flexible or at least partially flexible, bendable or malleable electrical conductors EC are disposed on or within first layer 112, or are disposed at least partially between first layer 112 and second layer 116. Each of flexible, bendable or malleable electrical conductors EC have at least first proximal and second distal ends (not shown in Figs. 4A through 4C, but shown in, for example, Fig. 6A).

Sensing electrodes E and corresponding overlying electrically conductive gel disks CG attached thereto are operably connected to corresponding ones of electrical conductors EC, which are configured to convey electrical signals sensed by sensing electrodes E and corresponding overlying electrically conductive gel disks CG to proximal electrical connections 115 (see, for example, Fig. 6A) configured for attachment to corresponding ECG mapping cable connectors 107, or to any other suitable electrical connector configured to convey electrical signals generated by sensing electrodes E to data acquisition device 210.

Adhesive members or disks AD surround at least partially corresponding ones of sensing electrodes E/CG, and are employed to attach or adhere the customizable electrophysiological mapping sensor patch to the patient's torso 14 after protective cover 116 has been removed by the user or health care provider.

Position markers PM have a position overlying and corresponding to that of at least selected ones, if not all, of sensing electrodes E/CG, and permit the precise position of the selected electrodes or each electrode E on the customizable electrophysiological mapping sensor patch to be ascertained quickly and easily using scanner or imaging system 300 in combination with PVM 400 (as described above). Position markers PM may include suitable MRI, CT and/or ultrasound contrast agents to enhance their detection, and increase the accuracy of determining their respective positions on the patient's torso 14.

In another embodiment, sensing electrodes E or portions attached thereto or forming a portion thereof function as position markers, and are configured to include suitable MRI, CT and/or ultrasound contrast agents therein or thereon so they may be detected easily by a suitable imaging system (e.g., an MRI, CT and/or ultrasound system). Fiducial markers may also be included in patches 101, 103 and/or 105, which are configured to operate in conjunction with an external imaging system, such as an MRI, CT and/or ultrasound system 300, and permit the precise spatial positions or locations of the fiducial markers to be determined using such imaging systems 300.

In one embodiment, electrical conductors EC are formed by printing or inking an electrically conductive and bendable or flexible material onto first layer 112, second layer 114, both layers, or other layers disposed therebetween. In one embodiment, first or top layer 112 comprises a flexible and/or stretchable layer comprising a woven textile comprising one or more elastomeric fiber yarns. Electrical conductors EC may be formed, by way of non-limiting example, by screen printing with a thickness of about 0.02 mm and a width of about 1 mm, by integrating electrically conductive yarns into layers 112, 114 or other layers, where the yarn has a diameter of about 0.05 mm Electrically conductive materials such as gold, silver, copper, carbon or metals or metal alloys may also be attached (for example by gluing or looming) to layer 112 or 114. Other embodiments of electrical conductors EC include electrical conductors comprising wires, cables, ribbon conductors, flexible electronic circuits, flex circuits, and flexible plastic substrates with electrical conductors disposed thereon or therein. Further embodiments of electrical conductors EC include electrical conductors comprising polyimide, PEEK or transparent conductive polyester film, and printed or screen printed flexible silver or other metal or metal alloy circuits disposed on polyester.

In one embodiment, the distal end of each electrical conductor EC is electrically and operably connected or attached to an electrode E having, by way of example, a diameter of about 14 mm. In one embodiment, electrical conductors EC and/or electrodes E have a thickness of about 0.1mm and are formed by screen printing or printing on a PET substrate having a thickness, for example, of 0.05 mm. PET is polyethylene terephthalate, a thermoplastic polymer resin of the polyester family suitable for use in in fibers for clothing layers. The PET layer or substrate may be glued or otherwise bonded to layer 112 or 114. The proximal end of each electrical conductor EC forms or is attached to proximal electrical connection or tab 115, which is configured for an external electrical connector such as a connector 107 of a mapping cable 102 to be connected thereto, for example by crimping. When formed by screen printing, electrically insulating or second layer 114 may have a thickness of about 0.02mm, and be formed, by way of example, from a material designated ELECTRODAG 452SS™ MANUFACTURED BY Henkel®, which in one embodiment is applied on a lower or bottom surface of layer 112 after electrical conductors EC have been printed or otherwise formed thereon so as to cover all electrically conductive components except electrodes E and electrical connections or tabs 115.

In some embodiments, adhesive members or rings AD have an internal diameter of about 14 mm, an outside diameter of about 24 mm, and a thickness of about 1mm. By way of example, adhesive members AD may be formed of polyethylene foam impregnated or coated with a synthetic rubber adhesive (for example, VANCIVE® MED5696R), and are glued or otherwise attached to electrically insulative layer 114 near each electrode E. The inner diameter of each adhesive member AD may be filled with a solid or compressible electrically conductive gel disc CG having, for example, an outer diameter of about 14 mm, a thickness of about 0.8 mm, where the gel comprises CONFORT® M807 manufactured by R&D Medical. In one embodiment, each electrically conductive gel disk or member CG is attached to underlying electrically conductive electrode E (as shown in Fig. 4B). In other embodiments, electrically conductive gel disk or member CG is attached directly to underlying electrical conductor EC. Adhesive members AD and solid gel discs CG may be covered by individual protective layers 116 or by a column, row or sheet forming a protective layer 116, where the protective layer may be peeled easily away from adhesive members AD and gel disks CG. By way of example, protective layer 116 may be formed of a polyester film having a thickness of 0.1mm such as KEMAFOIL® HSPL100 manufactured by COVEME®. Layer 116 may be configured to help preserve the adhesive and compressible properties of adhesive members AD and gel disks CG. On the opposite side of first or top layer 112, in front of each solid gel disc CG or electrode E and electrical conductor EC there is positioned a position marker PM, which in one embodiment is a dome-shaped member encapsulating an MRI and CT compatible contrast agent or product, such as fish oil (vitamin E), or any other suitable CT or MRT/MRI contrast or image enhancement medium or agent, which aids in providing a precise position for the spatial position of each electrode E.

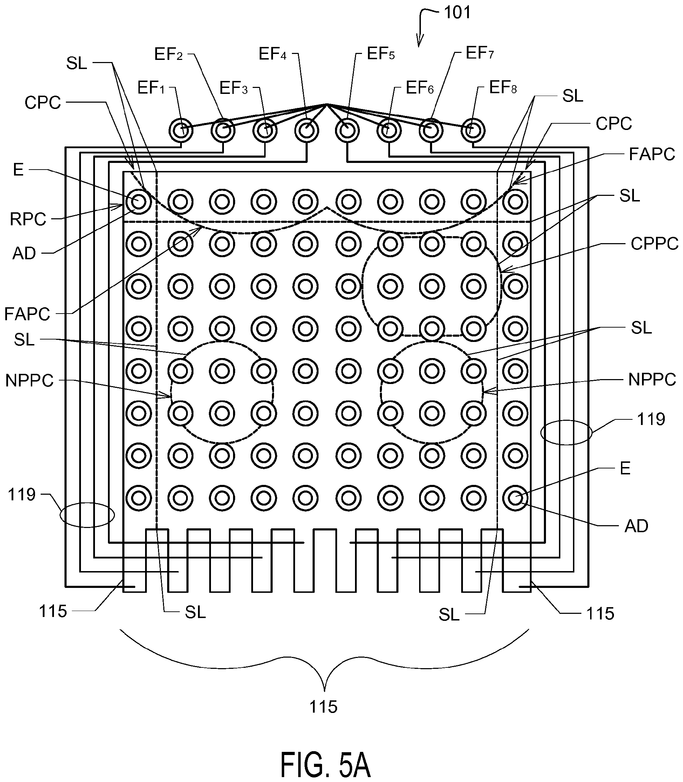

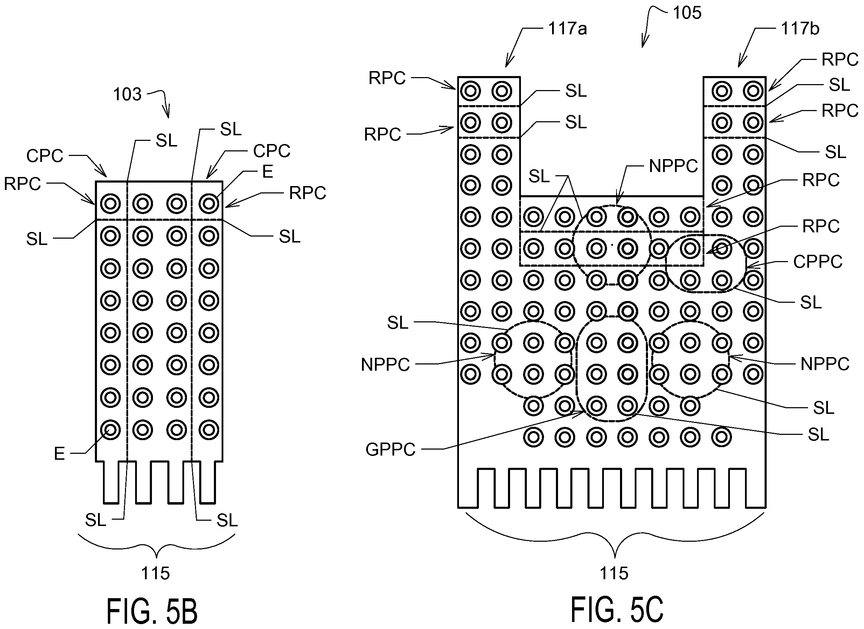

Referring now to Figs. 5A through 5C, there are shown embodiments of a customizable electrophysiological mapping sensor front patch 101 (Fig. 5A), a customizable electrophysiological mapping sensor side patch 103 (Fig. 5B), and a customizable electrophysiological mapping sensor rear patch 105 (Fig. 5C). Patches 101, 103 and 105 are customizable because various pre-cuts may be removed therefrom or partially removed therefrom according to: (a) the type of medical procedure that is to be carried out on a particular patient (e.g., electrophysiological mapping of a patient's cardiac electrical activity); (b) the types of combined medical procedures that are to be carried out on a particular patient (e.g., electrophysiological mapping of a patient's cardiac electrical activity combined with high intensity focused ultrasound (HIFU) ablation of the patient's heart); (c) the types of devices or components that are to be attached or secured to patches 101, 103 and 105,and (d) the physical dimensions and other characteristics of a particular patient (e.g., chest dimensions, waist size, torso dimensions, breast dimensions, etc.).

Continuing to refer to Figs. 5A through 5C, score lines SL are disposed in front mapping sensor patch 101, side mapping sensor patch, and rear mapping sensor patch 105, and define pre-cut portions thereof, including column pre-cuts CPC, row pre-cuts RPC, female anatomy pre-cut FAPC, cardioversion patch pre-cut CPCC, navigation patch pre-cuts NPCC, and an anatomical reference mark pre-cut APC (not shown in the Figures).

Each pre-cut is configured and shaped for a specific purpose and application. For example, row and column pre-cuts are shaped and configured to permit the health care provider or user to customize the width or height of a patch 101, 103 or 105 to match the requirements set by the dimensions and configurations of a particular patient's torso 14.

Column pre-cuts CPC in patches 101, 103 and 105 may be employed to customize the width of such patches according to a patient's torso and other dimensions. Smaller patients may require reduced widths for such patches.

Row pre-cuts RPC in patches 101, 103 and 105 may be employed to customize the height of such patches according to a patient's torso and other dimensions. Smaller patients may require reduced heights for such patches. In rear patch 105 shown in Fig. 5C, shoulder patch length may be shortened by removing one or more of the RPCs located on either side of and at the top of rear patch 105. A CPC may also be included on either or both sides of rear patch 105 (not shown in Fig. 5C).

Female anatomy pre-cut FAPC is shaped and configured to permit the health care provider or user to customize a front patch 101 to permit a female patient's breasts to fit over or along the upper edge of patch 101; female anatomy electrodes EF₁ - EF₈ are configured to replace the electrodes lost by removing pre-cut FAPC, and are attached directly to the patient's breasts. Other separate patches and electrodes, located apart from patches 101, 103 and 105 may also be included in MES 100.

Pre-cuts NPCC (navigation pre-cut), CPCC (cardioversion pre-cut), and GPPC (ground patch pre-cut -- see Fig. 5C) are configured and shaped to accept within the boundaries thereof a corresponding transducer, sensor or electrode.

In one embodiment, and when removed from front patch 101, anatomical reference mark pre-cut APC is a hole through which a health care provider or user may place his or her finger to locate a patient's xiphoid process (a bony landmark and protrusion from a patient's sternum), and then to appropriately position front patch 101 with APC located directly over the patient's xiphoid process. Such a patch registration process on the patient's torso 14 can help position all of patches 101, 103 and 105 in optimum and appropriate positions on the patient's body.

Any of the pre-cut types described above may be included in any of the patch types (i.e., front, side or rear patches). In addition, patch types may be combined into a single unitary patch (e.g., combined front and side patches, combined side and rear patches, etc.). Other specific types of pre-cuts are also contemplated, such as pre-cuts for ultrasonic transducers and sensors, diagnostic sensors or transducers, and the like, which may be configured and shaped according to the particular function a given pre-cut is to serve, and/or the device with which it is intended to be used.

In addition, any of patches 101, 103 and 105 may further comprise an anchor, an attachment or another device or devices attached or secured thereto such as adhesive patches, VELCRO® patches, plastic or metal hooks, loops, substrates, sockets, snaps, snap caps, zippers, posts, studs, which, by way of non-limiting example, may be employed to hold sensors or transducers in a desired position, route cables or wires, or otherwise hold or anchor assorted devices, components or materials in place. Such sensors and transducers may include ultrasound sensors and transducers, such as HIFU transducers and HIFU imaging probes, and may also include transmitters and/or receivers configured to permit the position of a catheter or portion(s) of a catheter within the patient's body to be determined, where the catheter includes electrode(s), sensor(s) or antennae configured to receive and/or transmit electromagnetic signals having frequencies outside the range of natural heart electrical signals, and which are received and/or transmitted from predetermined or known location(s) on the catheter body (e.g., near or at the distal tip of the catheter). In some embodiments, pre-cuts and/or anchors, attachments or other devices attached or secured to patches 101, 103 and 105 are labelled, marked, or colored-coded according to function or type so as to facilitate ease of use and reduce user or operator error.

In one embodiment, score lines SL in front patch 101, side patch 103 and rear patch 105 are configured to permit a user to remove or partially remove desired pre-cut portions from the patch by the user pulling or cutting the patch and/or the desired pre-cut portion along or near the corresponding score line SL. In one embodiment, score lines SL are formed in one or more of layers 112, 114, and 116, as well as in portions of the electrical conductors EC that are traversed by the score lines SL. In one embodiment, score lines SL comprise adjoining through-spaces or perforations that penetrate through the top, middle and/or bottom layers of an electrophysiological mapping sensor patch so that the cut-out defined thereby may be easily torn or cut along the score line SL, and thus be removed from the surrounding or adjoining patch by a user or health care provider. Score lines SL and their corresponding perforations are configured according to the thickness and composition of the material or materials through which they are formed so they may be torn reasonably uniformly along the score line SL (or tear line) by a user or health care provider once the tear has been initiated. Where a score line SL traverses an electrical conductor EC, more closely spaced perforations may be required in the traversed electrical conductor EC to permit the score line SL to be torn through the electrical conductor EC without undue effort. Score lines SL need not be straight, but may be curved, waved or otherwise shaped. Score lines SL may be formed using a cutting knife, blade or roller, a laser, a laser scoring machine, a die and punch, or any other suitable apparatus or method.

In one alternative embodiment, one or more removable pre-cuts included in patches 101, 103 and 105 may be adhered to patch 101, 103 and/or 105 by means of a suitable adhesive disposed, for example, around the periphery of each pre-cut, thereby eliminating or reducing the need for perforations or score lines SL. Such an adhesive has sufficient strength to hold each pre-cut in place, but is also weak enough to permit each pre-cut to be peeled away and removed from the patch by a user or health care provider. Zippers or other types of separable or separating devices may also be used to form the boundaries of removable pre-cuts.

Fig. 6A shows one embodiment of a customizable electrophysiological mapping sensor front patch 101, which for illustrative purposes, and to better illustrate one embodiment of how electrical conductors EC may be disposed inside patch 101, which for illustrative purposes is shown in Fig. 6A as having top or first layer 112 removed therefrom. Each of electrical conductors EC is operably connected at its distal end to one of electrodes E₁ through Eso, and is connected electrically at its proximal end to a corresponding one of proximal electrical connection or tab 115a through 115j. In one embodiment, each electrical conductor EC is electrically isolated or insulated from adjoining electrical conductors EC, although in other embodiments multiple electrical conductors EC may be joined or wired in parallel or series according to the specific design or functional objectives at hand (e.g., providing electrical conductor redundancy in patch 101, 103 and/or 105, measuring electrical signals in parallel with multiple electrodes E wired in parallel, etc.). Some electrical conductors EC shown in Fig. 6A traverse pre-cuts NPCC and CPCC. In the embodiment shown in Fig. 6A, removal of such pre-cuts from front patch 101 results in rendering not only electrodes E disposed within such pre-cuts inoperative (since they are removed entirely along with the corresponding pre-cut when removed from front patch 101), but also rendering inoperative some electrodes E disposed above or distally from the removed pre-cut since the electrical conductors EC corresponding thereto are terminated or trimmed when the pre-cut is removed. For example, removal of pre-cut NPCC disposed on the left side of Fig. 6A would result in electrodes E₉ through E₁₂ being rendered inoperative (in addition to electrodes E₅ and E₁₃ disposed within the boundaries of pre-cut NPPC also being rendered inoperative).

Thus, in some embodiments patches 101, 103 and/or 105 are configured such that with respect to electrodes E that are disposed distally from and above, but not within, the boundaries of a pre-cut, electrical conductors EC corresponding thereto are routed around or outside the boundaries of such pre-cuts such that when the pre-cut is removed an electrical connection is maintained between the distally located electrode E and its corresponding proximal electrical connection or tab 115. In such embodiments, electrical conductors EC corresponding to electrodes E located distally from and directly above the pre-cuts may be routed around the pre-cuts, and in being so configured may even cross over adjoining or other electrical conductors EC (provided they are electrically insulated therefrom at the crossover points).

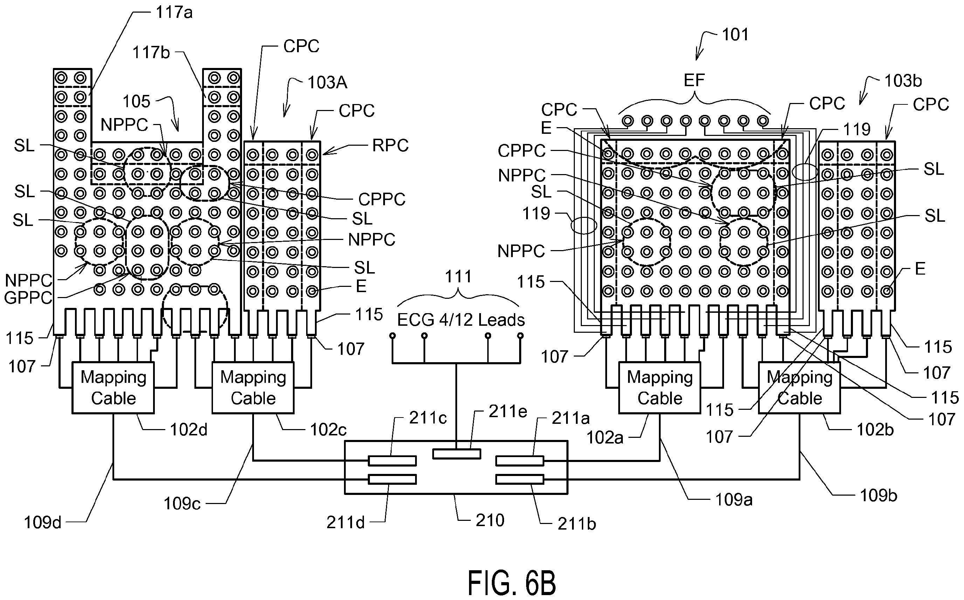

Fig. 6B shows one schematic embodiment of a customizable electrophysiological mapping sensor patch system comprising front patch 101, side patches 103A and 103B, and rear patch 105 operably connected to data acquisition device 210 through ECG mapping cables 102a through 102d. ECG 4/12 leads are configured for attachment to the patient, and are also shown as being connected to data acquisition device 210. Other electrodes can also be attached to the patient and data acquisition device 210, including as described above.

According to one embodiment, there is provided a method of configuring a customizable electrophysiological mapping sensor patch for a patient's torso, the mapping sensor patch comprising a first layer comprising at least a first flexible material, a second electrically insulative layer comprising at least a second flexible material, a plurality of flexible electrical conductors disposed on or within the first layer, or disposed at least partially between the first layer and the second layer, each of the flexible electrical conductors having at least first proximal and second distal ends, a plurality of sensing electrodes, each sensing electrode being operably connected to one of the electrical conductors, a plurality of adhesive members, each adhesive member surrounding at least partially a corresponding one or more of the sensing electrodes, at least one score line disposed in the mapping sensor patch, the score line defining at least one pre-cut portion of the mapping sensor patch, the score line being configured to permit a user to remove the at least one pre-cut portion from the mapping sensor patch by pulling or cutting the mapping sensor patch or pre-cut portion along or near the score line, where the method comprises removing at least one of the pre-cut portions from the mapping sensor patch on the basis of a procedure that is to be performed on the patient and on the basis of the patient's torso anatomy or torso dimensions.

According to another method and embodiment, and by way of illustrative example, customizable patches 101, 103 and 105 are employed in combination with an Amycard 01C electrophysiological mapping system using the following steps:
- A nurse begins proper skin preparation (shaving, scrubbing) on the patient according to hospital protocol.
- Front patch: As required or desired, the nurse removes one or more navigation patch pre-cuts, cardioversion patch pre-cuts, the female anatomy pre-cut, one or more row pre-cuts, and actuator or sensor pre-cuts by pulling apart the fabric along score lines SL in order to match patient gender and morphology requirements, and to take into account the interventional or sensing tools that are to be used (*e.g*., navigation patches, actuators and sensors, cardioversion patches, etc.).
- The nurse removes protective layer 116 from front patch 101 and places front patch 101 on the patient's chest using the anatomical reference mark. If the patient is a female, the nurse places the additional electrodes EF for female anatomy customization on the patient's chest.
- The nurse places the navigation patches and the cardioversion patches on the patient's torso within the front patch 101 in their proper designated areas.
- The nurse fastens one or more HIFU actuators (*i.e*., HIFU transducers or ablation signal sources) and HIFU receiving sensors or imaging probes on patch 101 using actuator and sensor anchors.
- Back patch 105: As required or desired, the nurse removes the navigation patch pre-cut, the cardioversion patch pre-cuts, the ground patch pre-cut, the row pre-cuts, the shoulder strap pre-cuts and the actuator and sensor pre-cuts by pulling apart the fabric along score lines SL in order to meet patient gender and morphology requirements, and to take into account the interventional or sensing tools that are to be used (*e.g*., navigation patches, actuators and sensors, cardioversion patches, etc.).
- The nurse removes protective layer 116 from rear patch 105 and places patch 105 on the patient's back and shoulders by aligning the shoulder straps with the top edge of the front patch 101.
- The nurse places the navigation patches and the cardioversion patches on the patient's torso within the rear patch 105 in their proper designated areas.
- The nurse fastens one or more HIFU actuators *(i.e.,* HIFU transducers or ablation signal sources) and HIFU receiving sensors or imaging probes on patch 105 using actuator and sensor anchors.
- Side Patch: As required or desired, the nurse removes row pre-cuts and column pre-cuts by pulling apart the fabric along perforated lines, along score lines SL in order to meet patient gender and morphology requirements.
- The nurse connects proximal connector tabs or connectors 115 located on patches 101, 103 and 105 to mapping cables 102.
- Electrophysiological mapping and HIFU ablation procedures are undertaken on the patient.

Note that the above method is merely illustrative, that some steps may not be included, and that other steps may be added.

According to some embodiments, and by way of example, the maximum physical dimensions of patches 101, 103 and 105 (without removing any pre-cuts), and the minimum physical dimensions of patches 101, 103 and 105 (after removing all pre-cuts) may be defined using the dimensions set forth in Table 1 below, where the maximum chest circumference is ensured by combining front patch 101, rear patch 105 and side patches 103a and 103b without any pre-cuts removed, and where maximum chest length is ensured by not removing any pre-cuts or electrodes from front patch 101. Customization of front patch 101 for patient sex is provided by the female anatomy pre-cut and corresponding separate electrodes EF.

**Table 1: Illustrative Customizable Patch Dimensions and Other Characteristics**

| Width (mm) | | Length (mm) | | Column Number of electrodes | | | | |
|---|---|---|---|---|---|---|---|---|
| | Max | Min | Max | Min | Max | Min | Max | Min |
| Front Patch | 320 | 260 | 240 | 210 | 10 | 8 | 56 | 80 |
| Rear Patch | 320 | | | 10 | 80 | 56 | | |
| Side Patch | 320 | 70 | | | 10 | 2 | 80 | 14 |

With respect to the prior art electrode configurations shown in Fig. 2A, some embodiments of patches 101, 103 and 105 provide the following advantages:
- Require the use of only 2-4 patches (vs. the 25-28 disposable electrodes or patient cables required in the prior art - an 85% reduction).
- Improve body morphology customization and adaptation (e.g., female patient customization)
- Permit the easy integration and use of cardioversion electrodes, ground electrodes, navigation sensors, transducers, etc.
- Permit the easy integration and use of HIFU probes
- Help ensure and optimize correct positioning of electrodes, sensors, patches, and transducers on the patient's torso.
- Reduce substantially the amount of time required to place electrophysiological mapping electrodes and other devices in accurate known positons on a patient's torso
- Improve the commercial attractiveness of non-invasive electrophysiological mapping systems as the ease and accuracy of positioning numerous electrophysiological mapping electrodes on a patient's torso is improved significantly.

In addition to the systems, devices, and components described above, it will now become clear to those skilled in the art that various methods associated therewith are also contemplated, such as methods of manufacturing and using customizable patches in accordance with the teachings of the present disclosure.

The foregoing outlines features of several embodiments so that those skilled in the art may better understand the detailed description set forth herein. Those skilled in the art will now understand that many different permutations, combinations and variations of patches 101, 103 and 105, and the various components, devices, systems and methods associated therewith, fall within the scope of the various embodiments. Those skilled in the art should appreciate that they may readily use the present disclosure as a basis for designing or modifying other processes and structures for carrying out the same purposes and/or achieving the same advantages of the embodiments introduced herein. Those skilled in the art should also realize that such equivalent constructions do not depart from the scope of the present disclosure, and that they may make various changes, substitutions and alterations herein without departing from the scope of the present disclosure as defined in the appended claims.

After having read and understood the present specification, those skilled in the art will now understand and appreciate that the various embodiments described herein provide solutions to longstanding problems in the use of electrophysiological mapping electrodes and systems, such as increasing the positional accuracy of electrodes and decreasing the amount of time required to deploy such electrodes on a patient in a clinical or hospital setting.

## Claims

1. A customizable electrophysiological mapping sensor patch (101, 103, 105) configured for placement on and attachment to a patient's torso (14), comprising:
a first substantially planar layer (112) comprising at least a first flexible material;
a second substantially planar electrically insulative layer (114) comprising at least a second flexible material;
a plurality of flexible electrical conductors (EC) disposed on or within the first layer, or disposed at least partially between the first layer and the second layer, each of the flexible electrical conductors having at least first proximal and second distal ends;
a plurality of sensing electrodes (E), each sensing electrode being operably connected to one of the electrical conductors;
a plurality of adhesive members (AD), each adhesive member surrounding at least partially a corresponding one or more of the sensing electrodes, and
at least one score line (SL) disposed in the mapping sensor patch, the score line defining at least one pre-cut portion of the mapping sensor patch including corresponding sensing electrodes, the score line being configured to permit a user to remove or partially remove the at least one pre-cut portion from the mapping sensor patch, thus removing the corresponding sensing electrodes, by pulling or cutting the mapping sensor patch or pre-cut portion along or near the score line;
**characterized in that** the at least one pre-cut portion of the patch comprises one or more of a row pre-cut (RPC), designed for removing a corresponding row of sensing electrodes to customize the height of the mapping sensor patch, one or more of a column pre-cut (CPC), designed for removing a corresponding column of sensing electrodes to customize the width of the mapping sensor patch, and one or more of: a cardioversion patch pre-cut (CPPC), a navigation patch pre-cut (NPPC), a ground patch pre-cut (GPPC), a female anatomy customization pre-cut (FAPC), an actuator pre-cut, a sensor pre-cut, or an anatomical reference mark pre-cut (APC).

2. The customizable electrophysiological mapping sensor patch of claim 1, further comprising a plurality of position markers (PM), each position marker having a position corresponding to that of a selected one of the sensing electrodes (E).

3. The customizable electrophysiological mapping sensor patch of any of the preceding claims, wherein at least one of the first layer (112) and the second layer (114) comprises a woven fabric.

4. The customizable electrophysiological mapping sensor patch of any of the preceding claims, wherein at least some of the first proximal ends of the electrical conductors (E) are shaped and configured for attachment to external mapping cable electrical connectors (115).

5. The customizable electrophysiological mapping sensor patch of any of the preceding claims, wherein at least some of the electrical conductors (EC) comprise a screen printed electrically conductive material disposed upon the first layer (112) or the second layer (114), or an electrically conductive fabric or yarn disposed upon or within the first layer or the second layer.

6. The customizable electrophysiological mapping sensor patch of any of the preceding claims, wherein at least some of the sensing electrodes (E) comprise a metal or metal alloy member, an electrically conductive yarn or fiber, a screen printed electrically conductive material, or a screen printed electrically conductive material incorporated into or forming a portion of the distal end of the electrical conductor (EC) corresponding thereto.

7. The customizable electrophysiological mapping sensor patch of any of the preceding claims, wherein at least some of the sensing electrodes (E) comprise an electrically conductive gel.

8. The customizable electrophysiological mapping sensor patch of any of the preceding claims, further comprising additional electrodes not attached to the first layer (112) or second layer (114) and configured for placement on one or more of a patient's breasts, to replace sensing electrodes (E) removed with the female anatomy customization pre-cut (FAPC).

9. The customizable electrophysiological mapping sensor patch of any of the preceding claims, wherein at least some of the sensing electrodes (E) comprise one or more magnetic resonance imaging (MRI), computed tomography (CT), and ultrasound contrast agents.

10. The customizable electrophysiological mapping sensor patch of any of the preceding claims, further comprising a protective layer (116) configured to cover at least the adhesive members (AD), the protective layer being configured to be removed from the mapping sensor patch (101, 103, 105) by a user before the mapping sensor patch is applied to the patient's torso (14).

11. The customizable electrophysiological mapping sensor patch of any of the preceding claims, wherein the patch is a front patch (101) and has a width ranging between about 260 mm and about 320 mm, a length ranging between about 200 mm and about 250 mm, and further comprises between 50 and 90 electrodes arranged in between 7 and 11 columns.

12. The customizable electrophysiological mapping sensor patch of any of claims 1 to 10, wherein the patch is a back patch (105) and has a width ranging between about 300 mm and about 340 mm, a length ranging between about 200 mm and about 250 mm, and further comprises shoulder straps and between 50 and 90 electrodes arranged in between 8 and 12 columns.

13. The customizable electrophysiological mapping sensor patch of any of claims 1 to 10, wherein the patch is a side patch (103) and has a width ranging between about 50 mm and about 350 mm, a length ranging between about 200 mm and about 250 mm, and further comprises between 10 and 90 electrodes arranged in between 1 and 12 columns.

14. A system of customizable electrophysiological mapping sensor patches (101, 103, 105) configured for placement on and attachment to a patient's torso (14), comprising: a front mapping sensor patch (101), a rear mapping sensor patch (105), and a side mapping sensor patch (103) for the right side of the patient's torso and for the left side of the patient's torso,
wherein each sensor patch is a mapping sensor patch according to any of the preceding claims.

15. The system of customizable electrophysiological mapping sensor patches of claim 14, wherein the front patch (101) has a width ranging between about 260 mm and about 320 mm, a length ranging between about 200 mm and about 250 mm, and further comprises between 50 and 90 electrodes arranged in between 7 and 11 columns, the rear patch (105) has a width ranging between about 300 mm and about 340 mm, a length ranging between about 200 mm and about 250 mm, and further comprises shoulder straps and between 50 and 90 electrodes arranged in between 8 and 12 columns, and each side patch (103) has a width ranging between about 50 mm and about 350 mm, a length ranging between about 200 mm and about 250 mm, and further comprises between 10 and 90 electrodes arranged in between 1 and 12 columns.

16. A method of configuring a customizable electrophysiological mapping sensor patch (101, 103, 105) for a patient's torso (14), the mapping sensor patch comprising a first layer (112) comprising at least a first flexible material, a second electrically insulative layer (114) comprising at least a second flexible material, a plurality of flexible electrical conductors (EC) disposed on or within the first layer, or disposed at least partially between the first layer and the second layer, each of the flexible electrical conductors having at least first proximal and second distal ends, a plurality of sensing electrodes (E), each sensing electrode being operably connected to one of the electrical conductors, a plurality of adhesive members (AD), each adhesive member surrounding at least partially a corresponding one or more of the sensing electrodes, at least one score line (SL) disposed in the mapping sensor patch, the score line defining at least one pre-cut portion of the mapping sensor patch including a number of corresponding sensing electrodes, the score line being configured to permit a user to remove or partially remove the at least one pre-cut portion from the mapping sensor patch, thus removing the corresponding sensing electrodes, by pulling or cutting the mapping sensor patch or pre-cut portion along or near the score line,
**characterized in that** the at least one pre-cut portion of the patch comprises one or more of a row pre-cut (RPC), designed for removing a corresponding row of sensing electrodes to customize the height of the mapping sensor patch, one or more of a column pre-cut (CPC), designed for removing a corresponding column of sensing electrodes to customize the width of the mapping sensor patch, and one or more of: a cardioversion patch pre-cut (CPPC), a navigation patch pre-cut (NPPC), a ground patch pre-cut (GPPC), a female anatomy customization pre-cut (FAPC), an actuator pre-cut, a sensor pre-cut, or an anatomical reference mark pre-cut (APC)
and **in that** the method further comprises:
removing at least one of the pre-cut portions from the mapping sensor patch on the basis of a procedure that is to be performed on the patient (12) and on the basis of the patient's torso anatomy or torso dimensions.

## Patentansprüche

1. Anpassbares elektrophysiologisches Mapping-Sensor-Patch (101, 103, 105), das zur Platzierung auf und Anbringung an einem Rumpf (14) eines Patienten konfiguriert ist, umfassend:
eine erste im Wesentlichen planare Schicht (112), die zumindest ein erstes flexibles Material umfasst;
eine zweite im Wesentlichen planare elektrisch isolierende Schicht (114), die zumindest ein zweites flexibles Material umfasst;
eine Mehrzahl flexibler elektrischer Leiter (EC), die auf oder innerhalb der ersten Schicht angeordnet sind oder zumindest teilweise zwischen der ersten Schicht und der zweiten Schicht angeordnet sind, wobei jeder der flexiblen elektrischen Leiter zumindest erste proximale und zweite distale Ende aufweist;
eine Mehrzahl von Erfassungselektroden (E), wobei jede Erfassungselektrode operabel mit einem der elektrischen Leiter verbunden ist;
eine Mehrzahl von Haftgliedern (AD), wobei jedes Haftglied zumindest teilweise eine entsprechende eine oder mehrere der Erfassungselektroden umgibt, und
zumindest eine Kerblinie (SL), die in dem Mapping-Sensor-Patch angeordnet ist, wobei die Kerblinie zumindest einen vorgeschnittenen Abschnitt des Mapping-Sensor-Patches einschließlich entsprechender Erfassungselektroden definiert, wobei die Kerblinie dahingehend konfiguriert ist, einem Benutzer zu erlauben, den zumindest einen vorgeschnittenen Abschnitt von dem Mapping-Sensor-Patch zu entfernen oder teilweise zu entfernen, wodurch die entsprechenden Erfassungselektroden entfernt werden, und zwar durch Ziehen oder Schneiden des Mapping-Sensor-Patches oder des vorgeschnittenen Abschnitts entlang oder nahe der Kerblinie;
**dadurch gekennzeichnet, dass** der zumindest eine vorgeschnittene Abschnitt des Patches einen oder mehrere von Reihenvorschnitt (RPC), der zum Entfernen einer entsprechenden Reihe von Erfassungselektroden ausgelegt ist, um die Höhe des Mapping-Sensor-Patches anzupassen, einen oder mehrere von Spaltenvorschnitt (CPC), der zum Entfernen einer entsprechenden Spalte von Erfassungselektroden ausgelegt ist, um die Breite des Mapping-Sensor-Patches anzupassen, und einen oder mehrere von: einem Kardioversion-Patch-Vorschnitt (CPPC), einem Navigation-Patch-Vorschnitt (NPPC), einem Masse-Patch-Vorschnitt (GPPC), einem Weibliche-Anatomie-Anpassung-Vorschnitt (FAPC), einem Aktorvorschnitt, einem Sensorvorschnitt oder einem Anatomische-Referenzmarke-Vorschnitt (APC) umfasst.

2. Anpassbares elektrophysiologisches Mapping-Sensor-Patch nach Anspruch 1, ferner umfassend eine Mehrzahl von Positionsmarkierungen (PM), wobei jede Positionsmarkierung eine Position aufweist, die der einer ausgewählten der Erfassungselektroden (E) entspricht.

3. Anpassbares elektrophysiologisches Mapping-Sensor-Patch nach einem der vorhergehenden Ansprüche, wobei zumindest eine der ersten Schicht (112) und der zweiten Schicht (114) ein Gewebe umfasst.

4. Anpassbares elektrophysiologisches Mapping-Sensor-Patch nach einem der vorhergehenden Ansprüche, wobei zumindest einige der ersten proximalen Enden der elektrischen Leiter (E) zur Anbringung an externen elektrischen Mapping-Kabelverbindern (115) geformt und konfiguriert sind.

5. Anpassbares elektrophysiologisches Mapping-Sensor-Patch nach einem der vorhergehenden Ansprüche, wobei zumindest einige der elektrischen Leiter (EC) ein siebgedrucktes elektrisch leitfähiges Material, das auf der ersten Schicht (112) oder der zweiten Schicht (114) angeordnet ist, oder einen elektrisch leitfähigen Stoff oder Garn umfassen, das auf oder innerhalb der ersten Schicht oder der zweiten Schicht angeordnet ist.

6. Anpassbares elektrophysiologisches Mapping-Sensor-Patch nach einem der vorhergehenden Ansprüche, wobei zumindest einige der Erfassungselektroden (E) ein Metall oder Metalllegierungsglied, ein elektrisch leitfähiges Garn oder Faser, ein siebgedrucktes elektrisch leitfähiges Material oder ein siebgedrucktes elektrisch leitfähiges Material umfassen, das in das distale Ende des elektrischen Leiters (EC) integriert ist oder einen Abschnitt davon bildet, das bzw. der diesem entspricht.

7. Anpassbares elektrophysiologisches Mapping-Sensor-Patch nach einem der vorhergehenden Ansprüche, wobei zumindest einige der Erfassungselektroden (E) ein elektrisch leitfähiges Gel umfassen.

8. Anpassbares elektrophysiologisches Mapping-Sensor-Patch nach einem der vorhergehenden Ansprüche, ferner umfassend zusätzliche Elektroden, die nicht an der ersten Schicht (112) oder der zweiten Schicht (114) angebracht sind und zur Platzierung auf einer oder mehreren Brüsten eines Patienten konfiguriert sind, um mit dem Weibliche-Anatomie-Anpassung-Vorschnitt (FAPC) entfernten Erfassungselektroden (E) zu ersetzen.

9. Anpassbares elektrophysiologisches Mapping-Sensor-Patch nach einem der vorhergehenden Ansprüche, wobei zumindest einige der Erfassungselektroden (E) eines oder mehrere von Magnetresonanztomographie (MRT), Computertomographie (CT) und Ultraschallkontrastmittel umfassen.

10. Anpassbares elektrophysiologisches Mapping-Sensor-Patch nach einem der vorhergehenden Ansprüche, ferner umfassend eine Schutzschicht (116), die konfiguriert ist, zumindest die Haftglieder (AD) abzudecken, wobei die Schutzschicht konfiguriert ist, von dem Mapping-Sensor-Patch (101, 103, 105) durch einen Benutzer entfernt zu werden, bevor das Mapping-Sensor-Patch auf den Rumpf (14) des Patienten aufgebracht wird.

11. Anpassbares elektrophysiologisches Mapping-Sensor-Patch nach einem der vorhergehenden Ansprüche, wobei das Patch ein Front-Patch (101) ist und eine Breite in einem Bereich zwischen etwa 260 mm und etwa 320 mm, eine Länge in einem Bereich zwischen etwa 200 mm und etwa 250 mm aufweist, und ferner zwischen 50 und 90 Elektroden umfasst, die in zwischen 7 bis 11 Spalten angeordnet sind.

12. Anpassbares elektrophysiologisches Mapping-Sensor-Patch nach einem der Ansprüche 1 bis 10, wobei das Patch ein Rücken-Patch (105) ist und eine Breite in einem Bereich zwischen etwa 300 mm und etwa 340 mm, eine Länge in einem Bereich zwischen etwa 200 mm und etwa 250 mm aufweist, und ferner Schultergurte und zwischen 50 und 90 Elektroden umfasst, die in zwischen 8 bis 12 Spalten angeordnet sind.

13. Anpassbares elektrophysiologisches Mapping-Sensor-Patch nach einem der Ansprüche 1 bis 10, wobei das Patch ein Seiten-Patch (103) ist und eine Breite in einem Bereich zwischen etwa 50 mm und etwa 350 mm, eine Länge in einem Bereich zwischen etwa 200 mm und etwa 250 mm aufweist und ferner zwischen 10 und 90 Elektroden umfasst, die in zwischen 1 und 12 Spalten angeordnet sind.

14. System anpassbarer elektrophysiologischer Mapping-Sensor-Patches (101, 103, 105), zur Platzierung auf und Anbringung an einem Rumpf (14) eines Patienten konfiguriert sind, umfassend: ein Front-Mapping-Sensor-Patch (101), ein Rücken-Mapping-Sensor-Patch (105) und ein Seiten-Mapping-Sensor-Patch (103) für die rechte Seite des Rumpfes eines Patienten und für die linke Seite des Rumpfes eines Patienten,
wobei jedes Sensor-Patch ein Mapping-Sensor-Patch nach einem der vorhergehenden Ansprüche ist.

15. System von anpassbaren elektrophysiologischen Mapping-Sensor-Patches nach Anspruch 14, wobei das Front-Patch (101) eine Breite in dem Bereich zwischen etwa 260 mm und etwa 320 mm, eine Länge in dem Bereich zwischen etwa 200 mm und etwa 250 mm aufweist und ferner zwischen 50 und 90 Elektroden umfasst, die in zwischen 7 und 11 Säulen angeordnet sind, wobei das Rücken-Patch (105) eine Breite in dem Bereich zwischen etwa 300 mm und etwa 340 mm, eine Länge in dem Bereich zwischen etwa 200 mm und etwa 250 mm aufweist und ferner Schultergurte und zwischen 50 und 90 Elektroden umfasst, die in zwischen 8 und 12 Spalten angeordnet sind, und wobei jedes Seiten-Patch (103) eine Breite in dem Bereich zwischen etwa 50 mm und etwa 350 mm, eine Länge in dem Bereich zwischen etwa 200 mm und etwa 250 mm aufweist und ferner zwischen 10 und 90 Elektroden umfasst, die in zwischen 1 bis 12 Spalten angeordnet sind.

16. Verfahren zum Konfigurieren eines anpassbaren elektrophysiologischen Mapping-Sensor-Patches (101, 103, 105) für einen Rumpf (14) eines Patienten, wobei das Mapping-Sensor-Patch umfasst: eine erste Schicht (112), die zumindest ein erstes flexibles Material umfasst, eine zweite elektrisch isolierende Schicht (114), die zumindest ein zweites flexibles Material umfasst, eine Mehrzahl flexibler elektrischer Leiter (EC), die auf oder innerhalb der ersten Schicht angeordnet sind oder zumindest teilweise zwischen der ersten Schicht und der zweiten Schicht angeordnet sind, wobei jeder der flexiblen elektrischen Leiter zumindest erste proximale und zweite distale Enden aufweist, eine Mehrzahl von Erfassungselektroden (E), wobei jede Erfassungselektrode operabel mit einem der elektrischen Leiter verbunden ist, eine Mehrzahl von Haftgliedern (AD), wobei jedes Haftglied zumindest teilweise eine entsprechende eine oder mehrere der Erfassungselektroden umgibt, zumindest eine Kerblinie (SL), die in dem Mapping-Sensor-Patch angeordnet ist, wobei die Kerblinie zumindest einen vorgeschnittenen Abschnitt des Mapping-Sensor-Patches einschließlich einer Anzahl entsprechender Erfassungselektroden definiert, wobei die Kerblinie dahingehend konfiguriert ist, einem Benutzer zu erlauben, den zumindest einen vorgeschnittenen Abschnitt von dem Mapping-Sensor-Patch zu entfernen oder teilweise zu entfernen, wodurch die entsprechenden Erfassungselektroden entfernt werden, und zwar durch Ziehen oder Schneiden des Mapping-Sensor-Patches oder des vorgeschnittenen Abschnitts entlang oder nahe der Kerblinie;
**dadurch gekennzeichnet, dass** der zumindest eine vorgeschnittene Abschnitt des Patches einen oder mehrere von Reihenvorschnitt (RPC), der zum Entfernen einer entsprechenden Reihe von Erfassungselektroden ausgelegt ist, um die Höhe des Mapping-Sensor-Patches anzupassen, einen oder mehrere von Spaltenvorschnitt (CPC), der zum Entfernen einer entsprechenden Spalte von Erfassungselektroden ausgelegt ist, um die Breite des Mapping-Sensor-Patches anzupassen, und einen oder mehrere von: einem Kardioversion-Patch-Vorschnitt (CPPC), einem Navigation-Patch-Vorschnitt (NPPC), einem Masse-Patch-Vorschnitt (GPPC), einem Weibliche-Anatomie-Anpassung-Vorschnitt (FAPC), einem Aktorvorschnitt, einem Sensorvorschnitt oder einem Anatomische-Referenzmarke-Vorschnitt (APC) umfasst,
und dadurch, dass das Verfahren ferner umfasst:
Entfernen zumindest eines der vorgeschnittenen Abschnitte von dem Mapping-Sensor-Patch auf Basis eines Eingriffs, der an dem Patienten (12) durchzuführen ist, und auf Basis der Rumpfanatomie oder der Rumpfabmessungen des Patienten.

## Revendications

1. Patch de capteur cartographique électrophysiologique personnalisable (101, 103, 105) configuré pour placement sur et fixation au torse d'un patient (14), comprenant :
une première couche sensiblement plane (112) comprenant au moins un premier matériau flexible ;
une deuxième couche électriquement isolante sensiblement plane (114) comprenant au moins un deuxième matériau flexible ;
une pluralité de conducteurs électriques (EC) flexibles disposés sur ou dans la première couche, ou disposés au moins partiellement entre la première couche et la deuxième couche, chacun des conducteurs électriques flexibles ayant au moins des premières extrémités proximales et deuxièmes extrémités distales ;
une pluralité d'électrodes de détection (E), chaque électrode de détection étant fonctionnellement connectée à l'un des conducteurs électriques ;
une pluralité d'éléments adhésifs (AD), chaque élément adhésif entourant au moins partiellement une ou plusieurs des électrodes de détection correspondantes, et
au moins une ligne de coupe (SL) disposée dans le patch de capteur cartographique, la ligne de coupe définissant au moins une partie prédécoupée du patch de capteur cartographique comprenant des électrodes de détection correspondantes, la ligne de coupe étant configurée pour permettre à un utilisateur d'enlever ou enlever partiellement l'au moins une partie prédécoupée du patch de capteur cartographique, de façon à enlever les électrodes de détection correspondantes, par traction ou découpe du patch de capteur cartographique ou d'une partie prédécoupée le long ou à proximité de la ligne de coupe ;
**caractérisé en ce que** l'au moins une partie prédécoupée du patch comprend l'un ou plusieurs parmi une rangée prédécoupée (RPC), conçue pour enlever une rangée d'électrodes de détection correspondante pour personnaliser la hauteur du patch de capteur cartographique, l'un ou plusieurs parmi une colonne prédécoupée (CPC), conçue pour enlever une colonne d'électrodes de détection correspondante pour personnaliser la largeur du patch de capteur cartographique, et l'un ou plusieurs parmi : un patch de cardioversion prédécoupé (CPPC), un patch de navigation prédécoupé (NPPC), un patch de mise à la terre prédécoupé (GPPC), une personnalisation d'anatomie féminine prédécoupée (FAPC), un actionneur prédécoupé, un capteur prédécoupé, ou un repère de référence anatomique prédécoupé (APC).

2. Patch de capteur cartographique électrophysiologique personnalisable selon la revendication 1, comprenant en outre une pluralité de marqueurs de position (PM), chaque marqueur de position ayant une position correspondant à celle de l'une sélectionnée des électrodes de détection (E).

3. Patch de capteur cartographique électrophysiologique personnalisable selon l'une quelconque des revendications précédentes, dans lequel au moins l'une parmi la première couche (112) et la deuxième couche (114) comprend un tissu tissé.

4. Patch de capteur cartographique électrophysiologique personnalisable selon l'une quelconque des revendications précédentes, dans lequel au moins certaines des premières extrémités proximales des conducteurs électriques (E) sont formées et configurées pour fixation à des connecteurs de câble électrique de cartographie externes (115).

5. Patch de capteur cartographique électrophysiologique personnalisable selon l'une quelconque des revendications précédentes, dans lequel au moins certains des conducteurs électriques (EC) comprennent un matériau électriquement conducteur sérigraphié disposé sur la première couche (112) ou la deuxième couche (114), ou un tissu ou fil électriquement conducteur disposé sur ou dans la première couche ou la deuxième couche.

6. Patch de capteur cartographique électrophysiologique personnalisable selon l'une quelconque des revendications précédentes, dans lequel au moins certaines des électrodes de détection (E) comprennent un élément métallique ou d'alliage métallique, un fil ou une fibre électriquement conducteur, un matériau électriquement conducteur sérigraphié, ou un matériau électriquement conducteur sérigraphié incorporé dans ou formant une partie de l'extrémité distale du conducteur électrique (EC) correspondant à celle-ci.

7. Patch de capteur cartographique électrophysiologique personnalisable selon l'une quelconque des revendications précédentes, dans lequel au moins certaines des électrodes de détection (E) comprend un gel électriquement conducteur.

8. Patch de capteur cartographique électrophysiologique personnalisable selon l'une quelconque des revendications précédentes, comprenant en outre des électrodes supplémentaires non fixées à la première couche (112) ou la deuxième couche (114) et configurées pour placement sur l'un ou plusieurs des seins d'un patient, pour remplacer des électrodes de détection (E) enlevées avec la personnalisation d'anatomie féminine prédécoupée (FAPC).

9. Patch de capteur cartographique électrophysiologique personnalisable selon l'une quelconque des revendications précédentes, dans lequel au moins certaines des électrodes de détection (E) comprennent un ou plusieurs agents d'imagerie par résonance magnétique (IRM), de tomodensitométrie (TDM) et de contraste échographique.

10. Patch de capteur cartographique électrophysiologique personnalisable selon l'une quelconque des revendications précédentes, comprenant en outre une couche de protection (116) configurée pour recouvrir au moins les éléments adhésifs (AD), la couche de protection étant configurée pour être retirée du patch de capteur cartographique (101, 103, 105) par un utilisateur avant que le patch de capteur cartographique soit appliqué sur le torse du patient (14).

11. Patch de capteur cartographique électrophysiologique personnalisable selon l'une quelconque des revendications précédentes, le patch étant un patch frontal (101) et ayant une largeur dans la plage comprise entre environ 260 mm et environ 320 mm, une longueur dans la plage comprise entre environ 200 mm et environ 250 mm, et comprenant en outre entre 50 et 90 électrodes agencées en entre 7 et 11 colonnes.

12. Patch de capteur cartographique électrophysiologique personnalisable selon l'une quelconque des revendications 1 à 10, le patch étant un patch arrière (105) et ayant une largeur dans la plage comprise entre environ 300 mm et environ 340 mm, une longueur dans la plage comprise entre environ 200 mm et environ 250 mm, et comprenant en outre des bandoulières et entre 50 et 90 électrodes agencées en entre 8 et 12 colonnes.

13. Patch de capteur cartographique électrophysiologique personnalisable selon l'une quelconque des revendications 1 à 10, le patch étant un patch latéral (103) et ayant une largeur dans la plage comprise entre environ 50 mm et environ 350 mm, une longueur dans la plage comprise entre environ 200 mm et environ 250 mm, et comprenant en outre entre 10 et 90 électrodes agencées en entre 1 et 12 colonnes.

14. Système de patchs de capteur cartographique électrophysiologique personnalisables (101, 103, 105) configuré pour placement sur et fixation au torse d'un patient (14), comprenant : un patch avant de capteur cartographique (101), un patch arrière de capteur cartographique (105), et un patch latéral de capteur cartographique (103) pour le côté droit du torse du patient et pour le côté gauche du torse du patient,
dans lequel chaque patch de capteur est un patch de capteur cartographique selon l'une quelconque des revendications précédentes.

15. Système de patchs de capteur cartographique électrophysiologique personnalisables selon la revendication 14, dans lequel le patch avant (101) a une largeur dans la plage comprise entre environ 260 mm et environ 320 mm, une longueur dans la plage comprise entre environ 200 mm et environ 250 mm, et comprend en outre entre 50 et 90 électrodes agencées en entre 7 et 11 colonnes, le patch arrière (105) a une largeur dans la plage comprise entre environ 300 mm et environ 340 mm, une longueur dans la plage comprise entre environ 200 mm et environ 250 mm, et comprend en outre des bandoulières et entre 50 et 90 électrodes agencées en entre 8 et 12 colonnes, et chaque patch latéral (103) a une largeur dans la plage comprise entre environ 50 mm et environ 350 mm, une longueur dans la plage comprise entre environ 200 mm et environ 250 mm, et comprend en outre entre 10 et 90 électrodes agencées en entre 1 et 12 colonnes.

16. Procédé de configuration d'un patch de capteur cartographique électrophysiologique personnalisable (101, 103, 105) pour un torse du patient (14), le patch de capteur cartographique comprenant une première couche (112) comprenant au moins un premier matériau flexible, une deuxième couche électriquement isolante (114) comprenant au moins un deuxième matériau flexible, une pluralité de conducteurs électriques flexibles (EC) disposés sur ou dans la première couche, ou disposés au moins partiellement entre la première couche et la deuxième couche, chacun des conducteurs électriques flexibles ayant au moins des premières et deuxième extrémités proximales et distales, une pluralité d'électrodes de détection (E), chaque électrode de détection étant fonctionnellement connectée à l'un des conducteurs électriques, une pluralité d'éléments adhésifs (AD), chaque élément adhésif entourant au moins partiellement l'une ou plusieurs des électrodes de détection correspondantes, au moins une ligne de coupe (SL) disposée dans le patch de capteur cartographique, la ligne de coupe définissant au moins une partie prédécoupée du patch de capteur cartographique comprenant une pluralité d'électrodes de détection correspondantes, la ligne de coupe étant configurée pour permettre à un utilisateur d'enlever ou partiellement enlever l'au moins une partie prédécoupée du patch de capteur cartographique, de façon à enlever les électrodes de détection correspondantes, par traction ou découpe du patch de capteur cartographique ou d'une partie prédécoupée le long ou à proximité de la ligne de coupe,
**caractérisé en ce que** l'au moins une partie prédécoupée du patch comprend l'un ou plusieurs parmi une rangée prédécoupé (RPC), conçue pour enlever une rangée d'électrodes de détection correspondante pour personnaliser la hauteur du patch de capteur cartographique, l'un ou plusieurs parmi une colonne prédécoupée (CPC), conçue pour enlever une colonne d'électrodes de détection correspondante pour personnaliser la largeur du patch de capteur cartographique, et l'un ou plusieurs de : un patch de cardioversion prédécoupé (CPPC), un patch de navigation prédécoupé (NPPC), un patch de mise à la terre prédécoupé (GPPC), une personnalisation d'anatomie féminine prédécoupée (FAPC), un actionneur prédécoupé, un capteur prédécoupé, ou un repère de référence anatomique prédécoupé (APC)
et **en ce que** le procédé comprend en outre :
le retrait d'au moins l'une des parties prédécoupées du patch de capteur cartographique sur la base d'une procédure qui doit être effectuée sur le patient (12) et sur la base de l'anatomie du torse ou des dimensions du torse du patient.
